# EUROPEAN PATENT SPECIFICATION

(11) **EP 2 911 645 B1**
(45) Date of publication and mention of the grant of the patent: **15.03.2023**
(21) Application number: 13767014.7
(22) Date of filing: 30.09.2013
(51) Int. Cl.: A61K 8/26, A61K 8/28, A61Q 15/00, A61K 8/02

(54) **METHOD FOR REDUCING PERSPIRATION**
VERFAHREN ZUR REDUKTION DER SCHWEISSBILDUNG
PROCÉDÉ PERMETTANT DE RÉDUIRE LA TRANSPIRATION

(30) Priority: 29.10.2012 EP 12190317; 29.10.2012 EP 12190316
(43) Date of publication of application: 02.09.2015
(73) Proprietor: Unilever IP Holdings B.V., 3013 AL Rotterdam (NL); Unilever Global IP Limited, Wirral, Merseyside CH62 4ZD (GB)
(72) Inventor: FRANKLIN, Kevin, Ronald, Wirral Merseyside CH63 3JW (GB)
(74) Representative: Whaley, Christopher
(86) International application number: PCT/EP2013/070359
(87) International publication number: WO 2014/067731

(56) References cited:
- EP-A1- 1 374 844
- EP-A2- 0 295 070
- GB-A- 880 261
- US-B1- 6 277 359
- US-B1- 6 342 210
- US-B1- 6 375 937

## Description

The present invention is in the field of cosmetic compositions, in particular antiperspirant compositions and their use in reducing perspiration.

A variety of antiperspirant compositions have been marketed for many years. They serve to reduce perspiration, particularly following application to the surface of the body. Such compositions are typically considered cosmetic products. The compositions are most commonly applied to the underarm regions of the human body.

Several forms of antiperspirant composition are currently available. Many consumers choose to use so-called "stick" compositions, where the antiperspirant active is typically embedded in a waxy carrier material. This product form is designed to be drawn along the surface of skin and to leave a deposit of the antiperspirant and carrier material thereupon. This product form has numerous disadvantages, including complexities of manufacture and packaging and, for some consumers, an unpleasant sensory experience on application.

Another form of antiperspirant composition commonly available is the "roll-on". This product form typically involves a solution of antiperspirant active being in contact with a rotatable ball, which is rolled across the surface of the body to apply the composition. This product form also has numerous disadvantages, including the fact that a significant amount of solubiliser, typically water, must be used and problems associated with the dispenser, including leakage and sticking of the roll-ball. In addition, there can be unpleasant sensory aspects upon application for some consumers.

A third form of antiperspirant composition commonly available is an aerosol. Such products are designed to spray antiperspirant active onto the surface of the body. This product form also has numerous disadvantages, including complexity of manufacture due to the (often highly flammable) volatile propellant that is required and lack of capture by the body of the spray directed towards it. In addition, there can be unpleasant sensory aspects upon application for some consumers, in particular, a cold sensation resulting from the volatile propellant used.

An unusual form of antiperspirant composition is described in WO 02/085321 A1 (Colgate, equivalent to US 6,342,210). In this publication, there is described a method of forming micronized antiperspirant salts involving the formation of a glass, the breaking up of said glass into particles, the suspension of said particles in a non-aqueous liquid, and the grinding of this mixture to form a suspension with particles having an average size of less than 200 microns.

The present invention represents a new mode of application of antiperspirant to the surface of the human body that overcomes many of the problems associated with those of the past.

Particular advantages of the present invention are reduced material usage, ease of manufacture, and ease of transport, due in part to excellent storage stability.

The antiperspirant compositions used in the present invention are extremely concentrated in their marketed state, that is to say, the state in which they are transported and sold to the consumer. This reduces transport costs and frees shelf space at point of sale.

The antiperspirant compositions used in the present invention are extremely simple in nature, that is to say, they have few essential components and are very easy to manufacture. Most notably, they require no "carrier" material other than a relatively small amount of water. This means that there is very little "nonfunctional" material in the composition, improving the environmental profile over prior art compositions such as sticks, that require substantial waxy carrier; aerosols, that require substantial volatile propellant; and roll-ons, that require substantial liquid carrier, typically water.

A further benefit of the present invention is the reduced need for complicated dispenser technology. It will be appreciated that stick compositions typically require a plastic tube with a push up platform; that roll-on compositions typically require a plastic reservoir with a roll-ball mounted on top; and that aerosols require a pressurised canister with release valve, and spray channel. Antiperspirant glass monoliths suitable for use in accordance with the present invention require very little, if any packaging.

As a result of the reduced need for packaging, the present invention reduces the overall need for space on storage, shipping, and shelf.

In a first aspect of the present invention, there is provided a method of reducing perspiration comprising the application to the surface on the human body of a solid antiperspirant monolith which is treated with water prior to or during said application, the solid antiperspirant glass monolith having a content of aluminium or aluminium-zirconium antiperspirant salt of from 50 to 90% by weight, a water content of from 5 to 40% by weight, characterised in that the monolith is a glass and has a minimum surface area of greater than 0.5 cm² and wherein the glass monolith is an amorphous solid state of matter allowing a degree of visible light transmission of greater than 5%.

Herein, application to the surface of the human body may be direct or indirect, i.e. the water-treated solid antiperspirant glass monolith may be placed directly into contact with skin or it may be applied to an intermediary substrate which is then used for application of the treatment to the skin.

Herein, application of solid antiperspirant glass monolith to the surface of the human body includes its application in a dissolved or partially dissolved form. Herein, treatment of the solid antiperspirant glass monolith with water prior to or during application to the surface on the human body may involve wetting it with water and/or forming a solution, especially an aqueous solution, from it and applying this solution to the surface on the human body.

Herein, when a solution from a solid antiperspirant glass monolith is applied to the surface of the human body, this may be accompanied with undissolved or partially undissolved antiperspirant material.

Herein, "glass" refers to a non-crystalline, i.e. amorphous, solid state of matter allowing a degree of visible light transmission of greater than 5%. The visible light transmission is preferably greater than 50%. Optical clarity and visible light transmission may be reduced if the glass contains water insoluble additives, such as water insoluble fragrances or emollient oils. Typical glasses are noncompressible when squeezed with the fingers and generally have a brittle quality, enabling them to be fractured into multiple pieces if struck with a hard object. Herein, visible light is that having a wavelength between 390 nm and 750 nm. Herein, the "surface area" refers to the total surface area for the item discussed. Herein, percentages given are by weight, unless otherwise indicated.

Herein, materials described as "water insoluble" should be understood to have a solubility of less 0.1 g/l at 25°C.

The content of aluminium or aluminium-zirconium antiperspirant salt in the antiperspirant glass monolith is from 50 to 90%, preferably from 55 to 85%, and more preferably from 60 to 80%.

The water content of the antiperspirant glass monolith is from 5 to 40%, preferably from 10 to 35%, and more preferably from 15 to 30%. Water content may be measured using a thermal balance, such as a Sartorius MA30, by heating a powdered sample to 120°C in auto heat time mode.

The content of organic material in the antiperspirant glass monolith is relatively limited, always being less than 45%, preferably being less than 30%, more preferably less 15% and most preferably less than 10%.

The antiperspirant glass monolith has a minimum surface area of greater than 0.5 cm² and preferably greater than 1 cm². It may have a surface area much greater than this, but the important point is that the antiperspirant glass monolith(s) can be easily seen by the unaided human eye and that it can be easily handled, if so required. In certain embodiments, the antiperspirant treatment involves numerous monoliths, more appropriately termed beads and having a surface area of from 0.5 cm² to 25 cm² and preferably from 1 to 15 cm². In alternative embodiments, the monolith may be quite large; in particular, it may be up to 150 or 200 cm². The larger sizes of monolith, such as from 50 to 200 cm², are suitable for direct application, with treatment with water involving the wetting of the monolith prior to or during its application to the surface of the human body.

The only essential components of antiperspirant glass monoliths used in the present invention are water and an aluminium or aluminium-zirconium antiperspirant salt. The aluminium or aluminium-zirconium antiperspirant salt may be selected from any of those known in the art. They are typically astringent salts. Preferred salts are halohydrate salts, such as chlorohydrates.

Particularly suitable aluminium antiperspirant salts are halohydrates defined by the general formula Al₂(OH)ₓQ_{y}.wH₂O in which Q represents chlorine, bromine or iodine, x is variable from 2 to 5 and x + y = 6 while wH₂O represents a variable amount of hydration. Especially effective aluminium halohydrate salts are known as activated aluminium chlorohydrates and are made by methods known in the art.

Particularly suitable aluminium-zirconium antiperspirant salts are complexes of a combination of aluminium halohydrates and zirconium chlorohydrates together with amino acids such as glycine, which are disclosed in US-A-3792068 (Luedders et al). Antiperspirant salts of this type generally contain aluminium, zirconium and chloride with an Al/Zr ratio in a range from 2 to 10, especially 2 to 6, an Al/Cl ratio from 2.1 to 0.9 and a variable amount of glycine.

Antiperspirant actives that are activated, that is to say, of enhanced efficacy are also suitable. Such activated salts may be prepared through heating in solution, in some instances in the presence of additives such as soluble calcium salts and amino acids as described in EP1104282 (Gillette).

The antiperspirant glass monolith is typically formed of or from a glassy solid produced by cooling and/or evaporation from a concentrated aqueous solution of aluminium or aluminium-zirconium antiperspirant salt at elevated temperature to a temperature and/or water concentration below which it solidifies. In general, the glassy solid is produced by both cooling and evaporative loss of water from the concentrated aqueous solution of aluminium or aluminium-zirconium antiperspirant salt.

Herein, references to a "concentrated" solution of aluminium or aluminium-zirconium antiperspirant salt at elevated temperature should be understood to refer to a solution of concentration suitable to deliver an antiperspirant glass having a content of aluminium or aluminium-zirconium antiperspirant salt of from 50 to 90% and a water content of from 5 to 40%. Typically, such solutions have an initial content of aluminium or aluminium-zirconium antiperspirant salt of from 50 to 80% and an initial water content of from 10 to 40%.

The method of manufacture of the antiperspirant glass monolith generally involves the preparation of a solution of aluminium or aluminium-zirconium antiperspirant salt at an initial concentration of from 50 to 80%. In addition, this solution typically has a water content of from 5 to 40% by weight. The solution is prepared at an elevated temperature, typically from 70-90°C, and poured into a mould whilst at a temperature still within this range. In a typical procedure, multiple moulds are employed. The solution is allowed to cool in the mould with evaporative loss of water, said loss of water typically being from 1 to 20% of the water initially present.

Optional agents may be introduced into the hot aqueous solution of aluminium or aluminium-zirconium antiperspirant salt and mixed in whilst it is still fluid. Examples of such optional agents include benefit agents such as colorant, fragrance, emulsifier, or emollient oil. Introduction of such an agent, in particular fragrance, followed by cooling and/or evaporative loss of water, represents a method of entrapping the agent within the antiperspirant glass monolith.

There is provided a method of entrapping a water insoluble benefit agent, such as a fragrance, in an antiperspirant glass monolith, said method comprising the preparation of a concentrated solution of aluminium or aluminium-zirconium antiperspirant salt at elevated temperature, the addition of the water-insoluble benefit agent to said solution, preferably with an emulsifier also added, the cooling of said solution with evaporative loss of water to give an antiperspirant glass monolith of aluminium or aluminium-zirconium antiperspirant salt content of from 50 to 90% by weight and a water content of from 5 to 40% by weight. This method may be used to prepare antiperspirant glass monoliths comprising from 0.01 to 10% by weight of benefit agent.

When a water-insoluble benefit agent and an emulsifier are both added, these components may be added separately or together. Typically they are added together, often in the form of a pre-formed oil-in-water emulsion.

Optional agents and in particular benefit agents may be introduced as aqueous solutions, when water soluble, or as pre-formed emulsions, such as an oil-in-water emulsion of emollient oil or fragrance. Such solutions or emulsions may, as described above, be introduced into the hot aqueous solution of aluminium or aluminium-zirconium antiperspirant salt and mixed in whilst it is still fluid.

A benefit agent frequently employed in glasses according to the present invention is a fragrance. Such fragrances become entrapped within the glass monolith on cooling the molten mixed. Preferably, they are incorporated with an emulsifier which can serve as a fragrance solubiliser. This can have the benefit of reducing any loss of optical clarity that the fragrance might otherwise cause. Such fragrances may be included at from 0.01 to 10%, preferably 0.1 to 5%, and more preferably from 0.25 to 3% by weight of the antiperspirant glass monolith.

Suitable fragrances may be selected from amongst those commonly used in cosmetics and toiletries.

Suitable emulsifiers may be selected according to the task they need to perform. Typically, the emulsifier will be of non-ionic in nature. Typically the emulsifier will be relatively hydrophilic,_having a HLB of at least 8. Suitable emulsifiers include PEG-hydrogenated castor oil, available from BASF in the Cremaphor RH and CO ranges. The emulsifiers may be included at from 0.01 to 10%, preferably 0.1 to 6%, and more preferably from 0.5 to 4% by weight of the antiperspirant glass monolith.

In certain embodiments, a preferred benefit agent for use in the present invention is an emollient oil. Such oils may be used at from 0.01 to 10%, preferably 0.1 to 8%, and more preferably from 0.25 to 5% by weight of the antiperspirant glass monolith. Suitable emollient oils include silicone oils, such as DC244, DC245, DC344 and DC345, ex Dow Corning; alkyl esters, such as PurSyn Ester 2E7 (neopentylglycol dihexanoate), ex Exxon-Mobil; benzoate esters such as Finsolv TN (Trade Mark), ex Finetex Inc.; hydrogenated polyalkenes, such as Panalane, ex Amoco and PureSyn PAO 2 (hydrogenated polydecene), ex Exxon-Mobil; PPG ethers, such as Fluid AP (PPG-14 butylether), ex Union Carbide; isopropyl palmitate; phenylsilicone; and isopropyl myristate.

In certain embodiments where enhanced sensory benefits are desired, a humectant, such a polyol, may be included in the antiperspirant glass monolith. Preferred humectants are glycerol, propylene glycol, and dipropylene glycol; glycerol is particularly preferred. Such humectants may be employed from 0.01 to 20%, preferably 0.1 to 10%, and more preferably from 0.5 to 5% by weight of the antiperspirant glass monolith.

In certain embodiments, the antiperspirant glass monolith is wetted with water during application to the surface on the human body. This may be conveniently done by applying water to the surface of the human body prior to applying the monolith. Alternatively, the water may already be present on the surface of the body in the form of sweat.

In certain embodiments, the antiperspirant glass monolith is wetted with water prior to application to the surface on the human body. The antiperspirant glass monolith may be wetted in any suitable manner. It may be wetted by direct application of water or by use of a wetted substrate, such as a damp cloth or sponge.

In certain embodiments, the antiperspirant glass monolith's treatment with water involves its dissolution prior to its application to the surface on the human body. In such embodiments, the aqueous solution formed from the antiperspirant glass monolith may be applied by any suitable means, for example, it may be applied directly as a spray or it may be applied using an intermediary substrate, such as a sponge or cloth wipe.

Packaging suitable for antiperspirant glass monoliths used in accordance with the present invention will tend to be dependent upon the size of the monolith(s). For monoliths of relatively large size (having at least one dimension greater than 5 cm), a moulded plastic holder may be suitable, enabling the application of the monolith (typically after wetting) to the surface of the body, without the user having to hold the monolith directly in hand.

For monoliths in the form of beads or tablets, a simple packaging device such as a jar may be appropriate or, for some markets, a blister pack may be used. Such blister packs are preferably made entirely of materials that are impervious to water, it being desirable to keep the enclosed beads or tablets free from extraneous water. Typically blister packs may contain from 4 to 20 beads or tablets. Each bead or tablet may be sufficient for a single application or may be sufficient for multiple applications.

In a particular embodiment, the monoliths are moulded into the form of beads or tablets and are packaged in a blister pack. One or more of the beads or tablets is subsequently removed from its blister pack and is dissolved in water to give an aqueous solution, the aqueous solution being applied to the surface of the human body to deliver an antiperspirancy benefit.

In a further embodiment, one or more of the beads or tablets is removed from its blister pack and is applied directly to the surface of the human body, typically by use of a re-usable applicator.

In a further embodiment, one or more of the beads or tablets is removed from its blister pack and is ground to a powder before being applied to the surface of the human body, typically by use of a moistened cloth, sponge or other moisture containing substrate.

In using the antiperspirant glass monoliths in accordance with the invention, the consumer may choose to make up an antiperspirant solution of concentration chosen to meet his or her own requirements. For example, if a mildly astringent solution is required, a relatively dilute solution might be prepared or for an antiperspirant solution delivering strong antiperspirancy, a more concentrated solution would be prepared. A key benefit of the invention is the flexibility given to the consumer in this regard.

In preparing antiperspirant solutions from the glass monoliths, the consumer would typically immerse the monolith(s) in water, optionally with other components, and await dissolution. Agitation of the mixture, by swirling or stirring, for example, might be used to speed dissolution, as might the use of hot water. Use of water at a temperature of 40°C or greater, or even 60°C or greater, can speed up dissolution significantly. Alternatively or additionally, dissolution may speeded by reducing the particle size of the monolith(s), by grinding, for example.

### Examples

The silicone moulds used in these examples were made using Silicone Plastique^{®} available from makeyourownmolds.com. Equal parts of the two components were combined and kneaded together in accordance with the manufacturers' instructions. The resulting material was rolled to make a flat sheet and then suitable embossing tools were pressed into the silicone to make indentations of the desired shape. The silicone was left to cure for one hour and then the embossing tools were removed to leave the mould ready for use.

### Example 1 -ACH Glass Hemispheres

120g of aluminium chlorohydrate powder (Microdry Superultrafine, *ex* Summit-Reheis, water content 15.7%) was weighed into a 500ml glass jar. 80g of water was added and the mixture shaken. The jar was capped and placed in an oven at 85°C for 4 hours. During this time, the mixture was periodically shaken. A clear pourable solution was obtained. The jar was removed from the oven and the contents immediately dispensed into silicone moulds with the aid of a pipette. The moulds were left at ambient temperature, open to the atmosphere, for 24 hours. Clear, non-tacky, hemispherical glass beads having a water content of 28.1% and a planar diameter of 8 mm were obtained (total surface area: 1.51 cm²).

The water contents of the aluminium chlorohydrate raw material and of the glass beads were measured using a Sartorius MA30 thermal balance, by heating powdered samples to 120°C (in auto heat time mode). This method was also used in measuring each of the water contents quoted in the following examples.

### Example 2 - ACH Glass Discs

The process of Example 1 was repeated with different silicone moulds. The glass beads obtained were disc-shaped, having a diameter of 11 mm and a depth of 3 mm (total surface area: 2.9 cm²).

In a further repeat of the process of Example 1, silicone moulds were used to produce disc-shaped glass beads having diameter of 25 mm and a depth of 5 mm (total surface area: 13.7 cm²).

### Example 3 - Coloured ACH Glass Beads

The process of Example 1 was repeated, except that 0.5g of Acid Red coloured dye solution (0.25% in water) was added to the ACH solution after it had been heated at 85°C for 4 hours. The mixture was shaken thoroughly and then heated for a further 15 minutes. Clear, non-tacky, pink, hemispherical glass beads having a planar diameter of 8cm were obtained (total surface area: 1.51 cm²).

The process was subsequently repeated using Acid Blue dye to produce pale green hemispherical glass beads and Acid Violet dye to produce blue hemispherical glass beads.

### Example 4 - ZAG Glass Beads

120g of aluminium zirconium tetrachlorohydratex glycine complex (Rezal 36GP ex Summit/Reheis, water content 11.1%) was weighed into a 500ml glass jar. 80g of water was added and the mixture shaken. The jar was capped and placed in an oven at 85°C for 4 hours. During this time, the mixture was periodically shaken. A clear pourable solution was obtained. The jar was removed from the oven and the contents immediately dispensed into silicone moulds with the aid of a pipette. The moulds were left at ambient temperature, open to the atmosphere for 24 hours. Clear, non-tacky, hemispherical glass beads having a water content of 15.5% and a planar diameter of 8mm were obtained (total surface area: 1.51 cm²).

### Example 5 - ZAG Glass Fragments

The process of Example 4 was repeated, except that the hot solution taken from the oven was poured into a flat silicone dish [20 cm diameter] and allowed to cool. Once the solution had solidified, it was broken by tapping with a hammer to give irregularly shaped clear glass fragments.

### Example 6 - ASCH Glass Beads

The process of Example 1 was repeated with aluminium sesquichlorohydrate powder (Reach 301, ex Summit, water content 14.6%) instead of the aluminium chlorohydrate. Clear, non-tacky, hemispherical glass beads having a water content of 25.2% and a planar diameter of 8 mm were obtained (total surface area: 1.51 cm²) were again obtained.

### Example 7 - Fragranced ACH Glass Beads

116g of aluminium chlorohydrate powder ( Microdry Superultrafine, ex Summit-Reheis, water content 15.7%, as example 1) was weighed into a 500ml glass jar. 6g of PEG-40 hydrogenated castor oil (Cremophor RH40, ex BASF) was added as a fragrance solubiliser, together with 74g of water, and the mixture was shaken. The jar was capped and placed in an oven at 85°C for 4 hours. During this time, the mixture was periodically shaken. A translucent pourable solution was obtained. 4g of fragrance was added to this solution and the mixture was shaken thoroughly and then heated for a further 15 minutes at 85°C. The jar was then removed from the oven, shaken thoroughly and immediately dispensed into moulds with the aid of a pipette. The mould was left at ambient temperature, open to the atmosphere for 24 hours. Clear to slightly translucent, non-tacky, hemispherical glass beads having a planar diameter of 8 mm were obtained (total surface area: 1.51 cm²).

Following storage of the beads within a lidded jar, a pleasant level of fragrance was perceived in the head space when the lid was removed.

### Example 8 - Fragrance and Coloured ACH Glass Beads

116g of aluminium chlorohydrate powder (Microdry Superultrafine - Summit-Reheis, water content 15.7%, as example 1) was weighed into a 500ml glass jar. 6g of PEG-40 hydrogenated castor oil (Cremophor RH40 ex BASF) was added as fragrance solubiliser, jar together with 74g of water, and the mixture was shaken. The jar was capped and placed in an oven at 85°C for 4 hours. During this time, the mixture was periodically shaken. A translucent pourable solution was obtained. 4g of fragrance and 0.5g of Acid Violet dye solution were added to the mixture. The mixture was shaken thoroughly and then heated for a further 15 minutes. The jar was removed from the oven, shaken thoroughly and then immediately dispensed into moulds with the aid of a pipette. The moulds were left at ambient temperature, open to the atmosphere, for 24 hours. Clear to slightly translucent, blue, non-tacky, hemispherical glass beads having a planar diameter of 8 mm were obtained (total surface area: 1.51 cm²).

Following storage of the hemispherical glass beads within a lidded jar, a pleasant level of fragrance was perceived in the head space when the lid was removed.

The process of Example 8 was repeated using 76g water, 4g Cremophor RH40, a different fragrance (4g), and different moulds. Clear to slightly translucent, bluegreen, non-tacky, rectangular cuboid glass beads measuring 15 mm x 15 mm x 8 mm were obtained (total surface area: 9.3 cm²).

Following storage of the rectangular cuboid glass beads within a lidded jar, a pleasant level of fragrance was perceived in the head space when the lid was removed.

### Example 9 - Fragrance and Coloured ZAG Glass Beads

The process of Example 8 was repeated with the ACH replaced by the ZAG used in Example 4. Clear, non-tacky, hemispherical glass beads having a planar diameter of 8 mm were obtained (total surface area: 1.51 cm²).

### Example 10 - Glass Beads with Humectant

The processes described above for the preparation of ACH glass beads may be repeated with 5% of the water replaced by a water soluble humectant such as glycerol. This can lead to improved sensory properties on application to the surface of the body. In the following example, 5% of water soluble humectant is incorporated.

120g of aluminium chlorohydrate powder (Microdry Superultrafine, ex Summit-Reheis, water content 15.7%) was weighed into a 500ml glass jar. 70g of water and 10g of glycerol were added and the mixture shaken. The jar was capped and placed in an oven at 85°C for 4 hours. During this time, the mixture was periodically shaken. A clear pourable solution was obtained. The jar was removed from the oven and the contents immediately dispensed into silicone moulds with the aid of a pipette. The moulds were left at ambient temperature, open to the atmosphere, for 24 hours. Clear, non-tacky, hemispherical glass beads having a planar diameter of 8 mm were obtained (total surface area: 1.51 cm²).

### Example 11 - Glass Beads with Emollient

The processes described above for the preparation of Examples 1 to 10 may be repeated with the inclusion of 5% of emollient oil, such as sunflower seed oil, together with a suitable level of emulsifier. In the following example, 3% of emollient is incorporated.

114g of aluminium zirconium tetrachlorohydrate glycine complex (Rezal 36GP ex Summit/Reheis, water content 11.1%) was weighed into a 500ml glass jar. 4g of Steareth 2 was added as a solubiliser for the emollient, together with 76g of water, and the mixture was shaken. The jar was capped and placed in an oven at 85°C for 4 hours. During this time, the mixture was periodically shaken. A translucent pourable solution was obtained. 6g of sunflower seed oil was added to this solution and the mixture was shaken thoroughly and then heated for a further 15 minutes at 85°C. The jar was then removed from the oven, shaken thoroughly and immediately dispensed into moulds with the aid of a pipette. The moulds were left at ambient temperature, open to the atmosphere for 24 hours. Translucent, non-tacky, hemispherical glass beads having a planar diameter of 8 mm were obtained (total surface area: 1.51 cm²).

### Example 12 - AACH Glass Beads

100g of aluminium chlorohydrate powder (Microdry Superultrafine - Summit-Reheis, water content 15.7%, as example 1), 10g of calcium chloride, and 31.4g glycine were weighed into a 500ml glass jar. 58.6g of water was added, and the mixture was shaken. The jar was capped and place in an oven at 85°C for 18 hours, the extended heating time allowing activation of the ACH by the calcium chloride and glycine. During this heating, the mixture was periodically shaken. A clear pourable solution was obtained. The jar was removed from the oven and immediately dispensed into moulds with the aid of a pipette. The moulds were left at ambient temperature, open to the atmosphere, for 24 hours. Clear, non-tacky, hemispherical glass beads having a planar diameter of 8mm were obtained (total surface area: 1.51 cm²).

### Storage Stability Tests

The glass beads of Examples 1, 3, 5, and 8 were put into glass jars which were capped and placed in an oven at 50°C for 8 hours. During this period, none of the glass beads were observed to change in physical state, i.e., they did not melt, dissolve, stick together, or loose clarity. Upon cooling back to room temperature, there were no visible changes to any of the samples.

## Claims

1. A method of reducing perspiration comprising the application to the surface on the human body of a solid antiperspirant glass monolith which is treated with water prior to or during said application, the solid antiperspirant monolith having a content of aluminium or aluminium-zirconium antiperspirant salt of from 50 to 90% by weight, a water content of from 5 to 40% by weight, **characterised in that** the monolith is a glass and has a minimum surface area of greater than 0.5 cm² and wherein the glass monolith is an amorphous solid state of matter allowing a degree of visible light transmission of greater than 5%.

2. A method according to claim 1, wherein the solid antiperspirant glass monolith is at least partially dissolved in water and the resulting solution is applied to the surface of the human body.

3. A method according to any of the preceding claims, wherein the monolith has a minimum surface area of greater than 1 cm².

4. A method according to any of the preceding claims, wherein the content of aluminium or aluminium-zirconium antiperspirant salt in the antiperspirant glass monolith is from 60 to 80% by weight.

5. A method according to any of the preceding claims, wherein the water content of the antiperspirant glass monolith is from 15 to 30% by weight.

6. A method according to any of the preceding claims, wherein the solid antiperspirant glass monolith comprises an entrapped benefit agent.

7. A method according to any of the preceding claims, wherein the solid antiperspirant glass monolith comprises a fragrance.

## Patentansprüche

1. Verfahren zur Reduktion der Schweißbildung, umfassend das Auftragen eines festen schweißhemmenden Glasmonoliths auf die Oberfläche des menschlichen Körpers, der vor oder während des Auftragens mit Wasser behandelt wird, wobei der feste schweißhemmende Monolith einen Gehalt an schweißhemmendem Aluminium- oder Aluminium-Zirkonium-Salz von 50 bis 90 Gewichts-%, einen Wassergehalt von 5 bis 40 Gewichts-% aufweist, **dadurch gekennzeichnet, dass** der Monolith ein Glas ist und einen minimalen Oberflächenbereich von größer als 0,5 cm² aufweist und wobei sich der Glasmonolith in einem amorphen festen Aggregatzustand befindet, der einen Grad der Durchlässigkeit sichtbaren Lichts von größer als 5% ermöglicht.

2. Verfahren nach Anspruch 1, wobei der feste schweißhemmende Glasmonolith mindestens teilweise in Wasser gelöst ist und die erhaltene Lösung auf die Oberfläche des menschlichen Körpers aufgetragen wird.

3. Verfahren nach irgendeinem der vorhergehenden Ansprüche, wobei der Monolith einen minimalen Oberflächenbereich von größer als 1 cm² aufweist.

4. Verfahren nach irgendeinem der vorhergehenden Ansprüche, wobei der Gehalt des schweißhemmenden Aluminium- oder Aluminium-Zirkonium-Salzes in dem schweißhemmenden Glasmonolithen 60 bis 80 Gewichts-% beträgt.

5. Verfahren nach irgendeinem der vorhergehenden Ansprüche, wobei der Wassergehalt des schweißhemmenden Glasmonolithen 15 bis 30 Gewichts-% beträgt.

6. Verfahren nach irgendeinem der vorhergehenden Ansprüche, wobei der feste schweißhemmende Glasmonolith ein eingeschlossenes Vorteilsmittel umfasst.

7. Verfahren nach irgendeinem der vorhergehenden Ansprüche, wobei der feste schweißhemmende Glasmonolith einen Duftstoff umfasst.

## Revendications

1. Procédé de réduction de la transpiration comprenant l'application sur la surface du corps humain d'un monolithe de verre antiperspirant solide qui est traité avec de l'eau avant ou pendant ladite application, le monolithe d'antiperspirant solide ayant une teneur en sel antiperspirant d'aluminium ou d'aluminium-zirconium de 50 à 90 % en masse, une teneur en eau de 5 à 40 % en masse, **caractérisé en ce que** le monolithe est un verre et présente une surface superficielle minimale supérieure à 0,5 cm² et dans lequel le monolithe de verre est un état de matière solide amorphe permettant un degré de transmission de lumière visible supérieur à 5 %.

2. Procédé selon la revendication 1, dans lequel le monolithe de verre antiperspirant solide est au moins partiellement dissout dans de l'eau et la solution résultante est appliquée sur la surface du corps humain.

3. Procédé selon l'une quelconque des revendications précédentes, dans lequel le monolithe présente une surface superficielle minimale supérieure à 1 cm².

4. Procédé selon l'une quelconque des revendications précédentes, dans lequel la teneur en sel antiperspirant d'aluminium ou aluminium-zirconium dans le monolithe de verre antiperspirant est de 60 à 80 % en masse.

5. Procédé selon l'une quelconque des revendications précédentes, dans lequel la teneur en eau du monolithe de verre antiperspirant est de 15 à 30 % en masse.

6. Procédé selon l'une quelconque des revendications précédentes, dans lequel le monolithe de verre antiperspirant solide comprend un agent bénéfique piégé.

7. Procédé selon l'une quelconque des revendications précédentes, dans lequel le monolithe de verre antiperspirant solide comprend un parfum.
